Europäisches Patentamt

European Patent Office

Office européen des brevets

(11) Veröffentlichungsnummer : **0 025 940**
**B1**

(12) **EUROPÄISCHE PATENTSCHRIFT**

(45) Veröffentlichungstag der Patentschrift :
**15.12.82**

(21) Anmeldenummer : **80105407.3**

(22) Anmeldetag : **10.09.80**

(51) Int. Cl.³ : **C 07 C 29/03, C 07 C 31/20,**
**C 07 C 31/27, C 07 C 59/105**

(54) Verfahren zur Hydroxylierung olefinisch ungesättigter Verbindungen.

(30) Priorität : **19.09.79 DE 2937831**

(43) Veröffentlichungstag der Anmeldung :
**01.04.81 (Patentblatt 81/13)**

(45) Bekanntmachung des Hinweises auf die Patenterteilung : **15.12.82 Patentblatt 82/50**

(84) Benannte Vertragsstaaten :
**AT BE CH FR GB IT LI NL SE**

(56) Entgegenhaltungen :
**FR A 1 047 126**
**GB A 634 118**
**US A 2 492 201**

(73) Patentinhaber : **Degussa Aktiengesellschaft**
**Weissfrauenstrasse 9**
**D-6000 Frankfurt am Main 1 (DE)**

**Henkel Kommanditgesellschaft auf Aktien**
**Postfach 1100 Henkelstrasse 67**
**D-4000 Düsseldorf 1 (DE)**

(72) Erfinder : **Käbisch, Gerhard, Dr.**
**Palmstrasse 1**
**D-7888 Rheinfelden (DE)**
Erfinder : **Malitius, Horst**
**Blümleacker 5**
**D-7888 Rheinfelden; (DE)**
Erfinder : **Raupach, Siegfried**
**Langentalstrasse 24**
**D-7888 Rheinfelden (DE)**
Erfinder : **Trübe, Rudolf**
**Ernst-Reuter-Strasse 22**
**D-7888 Rheinfelden (DE)**
Erfinder : **Wittmann, Hans**
**Katzenbuckelweg 12**
**D-7888 Rheinfelden (DE)**

## Verfahren zur Hydroxylierung olefinisch ungesättigter Verbindungen

Die Erfindung betrifft ein Verfahren zur Hydroxylierung olefinisch ungesättigter Verbindungen mit 4 bis 36 Kohlenstoffatomen und über 40 °C liegenden Siedepunkten durch Umsetzung mit Ameisensäure und Wasserstoffperoxid bei erhöhter Temperatur und unter inniger Durchmischung und anschliessende Verseifung der enthaltenen Formiate.

Es ist bereits bekannt, 1,2-Diole durch Umsetzung von 1-Olefinen mit 8 bis 18 Kohlenstoffatomen mit Ameisensäure und Wasserstoffperoxid bei einer Temperatur von 40 °C und unter inniger Durchmischung und anschliessende Verseifung der gebildeten Formiate herzustellen (D. Swern et al. in J. Am. Chem. Soc. *68* (1946), Seiten 1 504 bis 1 507). Bei dem Bekannten Verfahren wird die Ameisensäure je nach dem eingesetzten Olefin in Mengen von etwa 9 bis etwa 31 Mol pro Mol Olefin eingesetzt und ein Wasserstoffperoxid mit einer Konzentration von 25,6 Gewichtsprozent verwendet. Die Umsetzung erfordert je nach dem eingesetzten Olefin eine Reaktionszeit zwischen etwa 8 und etwa 24 Stunden. Die Rohen 1,2-Diole fallen zwar bei der Verseifung der gebildeten Diolmonoformiate in Ausbeuten von 70 % und darüber an, die Ausbeute an reinem 1,2-Diol beträgt jedoch je nach dem eingesetzten Olefin nur zwischen 40 und 70 %.

Das erfindungsgemässe Verfahren ist nun dadurch gekennzeichnet, dass man die Ameisensäure in Mengen von 2 bis 6 Mol pro Mol Doppelbindung einsetzt, Wasserstoffperoxid mit einer Konzentration von 35 bis 98 Gewichtsprozent verwendet und die Umsetzung bei einer Temperatur zwischen 40 und 80 °C vornimmt.

Unter diesen Bedingungen genügt schon eine Reaktionszeit von weniger als 7 Stunden, beispielsweise von 1 bis 3 Stunden, zur Erzielung eines hohen Umsatzes. Die Ausbeute an reinem Diol liegt je nach dem eingesetzten Olefin zwischen etwa 87 und etwa 92 %.

Das erfindungsgemässe Verfahren ist mit etwa gleichem Erfolg auf lineare Olefine mit endständiger oder innenständiger Doppelbindung und auf cyclische Olefine anwendbar. Gegebenenfalls können die einzusetzenden Olefine auch verzweigt und/oder durch funktionelle Gruppen, beispielsweise Carboxylgruppen, substituiert sein. Sie können als reine Substanzen oder als Gemische von Homologen oder Isomeren eingesetzt werden. Da beim erfindungsgemässen Verfahren eine drucklose Arbeitsweise bevorzugt wird, sollen die Siedepunkte der einzusetzenden Olefine über 40 °C liegen. Besonders geeignet ist das erfindungsgemässe Verfahren zur Hydroxylierung längerkettiger linearer Olefine mit 8 bis 36 Kohlenstoffatomen und einer endständigen oder innenständigen Doppelbindung.

Monoolefine mit endständiger Doppelbindung, sogenannte α-Olefine, werden üblicherweise nach dem Ziegler-Äthylen-Oligomerisationsverfahren gewonnen. Dabei entstehen in der Hauptsache geradkettige Olefine mit einer geraden Kohlenstoffzahl. Bis zu einer Kohlenstoffzahl von etwa 18 sind sie im Handel als reine Substanzen erhältlich. Die noch längerkettigen Olefine erhält man im Handel normalerweise nicht mehr als reine Substanzen, sondern als Olefingemische, beispielsweise im Kettenlängenbereich $C_{20-24}$, $C_{24-28}$ oder $C_{30-}$. Monoolefine mit innenständiger Doppelbindung werden technisch durch Dehydrierung von Paraffin-Fraktionen gewonnen und stellen deshalb normalerweise entsprechende Gemische, z.B. mit 11 bis 14 Kohlenstoffatomen, dar, bei denen die Doppelbindungen statistisch verteilt sind.

Beim erfindungsgemässen Verfahren kann das Wasserstoffperoxid mit einer Konzentration von 35 bis 98 Gewichtsprozent, vorzugsweise mit einer Konzentration von 50 bis 90 Gewichtsprozent, eingesetzt werden. Vorteilhaft ist es, wenn man nicht von Anfang an die ganze Menge an Wasserstoffperoxid zusetzt, sondern vielmehr ein Gemisch aus Olefin und der Ameisensäure vorlegt und das Wasserstoffperoxid im Verlaufe von 1 bis 4 Stunden zudosiert. Die Zugabe des Wasserstoffperoxids kann kontinuierlich oder portionsweise erfolgen. Die allmähliche Zudosierung des Wasserstoffperoxids ist aus Sicherheitsgründen insbesondere dann zu empfehlen, wenn es in einer höheren Konzentration, beispielsweise einer solchen von über 50 Gewichtsprozent, eingesetzt wird.

Die Umsetzung zwischen dem Olefin und der in situ gebildeten Perameisensäure wird bei einer Temperatur zwischen 40 und 80 °C, vorzugsweise zwischen 50 und 70 °C, vorgenommen.

Zur Erzielung einer hohen Ausbeute reicht es zwar aus, wenn das Wasserstoffperoxid in einer Menge von einem Mol pro Mol Doppelbindung eingesetzt wird. Sehr hohe Umsätze, verbunden mit guten Ausbeuten, können jedoch erzielt werden, wenn das Wasserstoffperoxid in einem molaren Überschuss von 5 bis 30 %, bezogen auf den Gehalt des eingesetzten Olefins an Doppelbindungen, verwendet wird.

Das Ende der Umsetzung zwischen dem Olefin und der in situ gebildeten Perameisensäure ist bei der Herstellung von Diolen mit weniger als 16 Kohlenstoffatomen im allgemeinen daran zu erkennen, dass das Reaktionsgemisch einphasig wird oder allenfalls noch eine geringe Trübung aufweist. Wenn das Wasserstoffperoxid allmählich zudosiert wird, ist dieser Zustand normalerweise schon unmittelbar nach dem Ende der Zudosierung erreicht. Bei der Herstellung von Diolen mit 16 oder mehr Kohlenstoffatomen bleibt das Reaktionsgemisch im allgemeinen zweiphasig.

Die jeweils bevorzugte Aufarbeitungsweise trägt diesem Unterschied Rechnung: Aus einphasigen Systemen wird Zweckmässigerweise zunächst die Ameisensäure und das Wasser bei vermindertem Druck abgetrieben. Zweiphasige Systeme werden vorteilhafterweise zuerst einmal einer Phasentrennung unterworfen. In beiden Fällen kann anschliessend die organische Phase ein- oder mehrmals bei erhöhter Temperatur mit Wasser gerührt und nach dem Abkühlen von der wässrigen Phase getrennt werden. Tritt keine Phasentrennung ein, weil das gebildete Diol in erheblichem Masse wasserlöslich ist, so wird das Diol mit einem geeigneten Lösungsmittel, beispielsweise Äthylacetat, extrahiert. Besonders reine Diole erhält man, wenn man die organische Phase mit alkalisch reagierenden Stoffen behandelt. So kann die organische Phase zunächst etwa 15 Minuten lang bei erhöhter Temperatur, beispielsweise bei 70 °C, mit soviel Natronlauge gerührt werden, dass die noch vorhandene bzw. die durch Verseifung der enthaltenen Diolmonoformiate freiwerdende Ameisensäure neutralisiert wird. Nach Abtrennung der wässrigen Phase bzw. Extraktion des gebildeten Diols kann dann mit heissem Wasser ein- oder mehrmals nachgewaschen werden, bis das rohe Diol bzw. der Extrakt frei von Alkali ist. Der Reinheitsgrad der so, gegebenenfalls nach dem Abdestillieren des Extraktionsmittels, gewonnenen Rohprodukte ist im allgemeinen bereits so hoch, dass sie für vielerlei Umsetzungen direkt weiterverwendet werden können. Sind in speziellen Fällen noch höhere Reinheitsgrade erwünscht, lassen sich die rohen Diole leicht durch an sich bekannte Massnahmen, wie fraktionierte Destillation unter vermindertem Druck oder Umkristallisation aus geeigneten Lösungsmitteln, beispielsweise aus Äthylacetat, in Reinstprodukte überführen.

Die wirtschaftlichen Vorteile des erfindungsgemässen Verfahrens gegenüber dem bekannten von Swern et al. bestehen unter anderem darin, dass

1. die erforderliche Reaktionszeit wesentlich kürzer ist ;

2. die Aufarbeitung des rohen Reaktionsgemisches infolge der Verwendung geringerer Mengen an Ameisensäure und konzentrierteren Wasserstoffperoxids merklich erleichtert wird, weil nur noch relativ geringe Mengen an wässriger Ameisensäure destillativ aus dem System entfernt werden müssen ; und

3. die Ausbeuten an reinem Diol beträchtlich höher sind.

Die Verwendung zusätzlicher Lösungsmittel bei der Umsetzung zwischen dem Olefin und der in situ gebildeten Perameisensäure ist nicht erforderlich, da bei den angewandten Reaktionstemperaturen sowohl die eingesetzten Olefine als auch die rohen Reaktionsprodukte flüssig sind. Bei der anschliessenden Aufarbeitung der rohen Reaktionsprodukte kann jedoch die Verwendung von zusätzlichen Lösungsmitteln in den Fällen von Vorteil sein, in denen die reinen Diole einen relativ hohen Schmelzpunkt haben. Die rohen Reaktionsprodukte können dann mit Lösungsmitteln verdünnt werden, die ein hohes Lösungsvermögen für die gebildeten Diole, aber nur eine geringe Löslichkeit in Wasser aufweisen und die gegen Verseifung beständig sind. Geeignete Lösungsmittel sind beispielsweise Kohlenwasserstoffe oder Alkohole.

Das erfindungsgemässe Verfahren soll durch die nachfolgenden Beispiele näher verdeutlicht werden :

Beispiel 1

In einem 4 l-Rührkolben mit aufgesetztem Rückflusskühler und Tropftrichter wurden bei einer Temperatur von 50 °C 560 g $\alpha$-$C_8$-Olefin (5 Mol) und 940 g 98 gewichtsprozentige Ameisensäure (20 Mol) vermischt. Im Verlauf von 2 Stunden wurden 340 g 60 gewichtsprozentiges Wasserstoffperoxid (6 Mol) zudosiert. Nach beendeter Zudosierung war das anfänglich heterogene System einphasig geworden. Die leichtflüchtigen Anteile (Wasser und Ameisensäure) wurden im Verlauf von einer halben Stunde im Wasserstrahlvakuum bei einer Sumpftemperatur bis zu 110 °C abgetrieben. Anschliessend wurde das Sumpfprodukt mit 2,6 l Wasser versetzt, 3 Stunden am Rückfluss gekocht, abgekühlt und von der wässrigen Phase abgetrennt. Nach dem Trocknen im Vakuum wurden 690 g Rohprodukt erhalten, das noch einen Monoformiatgehalt von 5,7 Gewichtsprozent aufwies. Durch Rühren mit 25 gewichtsprozentiger Natronlauge (15 Minuten bei 70 °C) wurde der Monoformiatgehalt auf 0 Gewichtsprozent abgesenkt. Nach Abtrennung der wässrigen Phase wurde das rohe Diol durch Destillation gereinigt. Die zwischen 135 und 138 °C (12 Torr) übergehende Fraktion wog 635 g, was einer Ausbeute an reinem Diol von 87,0 % der Theorie entsprach. Der Schmelzpunkt des reinen Diols betrug 35 °C.

Zum Vergleich lässt sich den Angaben von Swern et al. entnehmen, dass aus $\alpha$-$C_8$-Olefin nach einer Reaktionszeit von 8 Stunden bei einer Temperatur von 40 °C und bei einem Molverhältnis von Olefin zu Ameisensäure von 1 : 9 das reine Diol mit einem Schmelzpunkt von 30-30,5 °C in einer Ausbeute von 58 % der Theorie erhalten wurde.

Beispiel 2

Das Beispiel 1 wurde wiederholt mit 840 g $\alpha$-$C_{12}$-Olefin (5 Mol) und 291 g 70 gewichtsprozentigem Wasserstoffperoxid (6 Mol) bei einer Reaktionstemperatur von 55 °C. Die Ausbeute an reinem Diol betrug 90,5 % der Theorie, der Schmelzpunkt des reinen Diols 61,5 °C.

Zum Vergleich lässt sich den Angaben von Swern et al. entnehmen, dass aus $\alpha$-$C_{12}$-Olefin nach einer Reaktionszeit von 24 Stunden bei einer Temperatur von 40 °C und bei einem Molverhältnis von Olefin zu Ameisensäure von 1 : 13 das reine Diol mit einem Schmelzpunkt von 60-61 °C in einer Ausbeute von 40 % der Theorie erhalten wurde.

Beispiel 3

Das Beispiel 1 wurde wiederholt mit 1 120 g $\alpha$-$C_{16}$-Olefin (5 Mol) und 240 g 85 gewichtsprozentigem Wasserstoffperoxid (6 Mol) bei einer Reaktionstemperatur von 60 °C. Die Ausbeute an reinem Diol betrug 92,3 % der Theorie, der Schmelzpunkt des reinen Diols 73,5 °C.

Zum Vergleich lässt sich den Angaben von Swern et al. entnehmen, dass aus $\alpha$-$C_{16}$-Olefin nach einer Reaktionszeit von 24 Stunden bei einer Temperatur von 40 °C und bei einem Molverhältnis von Olefin zu Ameisensäure von 1 : 31 das reine Diol mit einem Schmelzpunkt von 75-76 °C in einer Ausbeute von 58 % der Theorie erhalten wurde.

Beispiel 4

Analog zu Beispiel 1 wurden in einer 1 l-Rührapparatur 175 g eines technischen Olefingemisches im Kettenlängenschnitt von 11 bis 14 Kohlenstoffatomen, bei dem die Doppelbindungen statistisch verteilt über die gesamte Kettenlänge lagen, (~ 1 Mol) und 188 g 98 gewichtsprozentige Ameisensäure (4 Mol) vermischt. Im Verlauf von 1 Stunde wurden 61 g 70 gewichtsprozentiges Wasserstoffperoxid (1,25 Mol) zudosiert. Danach wurden Ameisensäure und Wasser unter vermindertem Druck abgetrieben und der Destillationssumpf 15 Minuten lang bei 70 °C mit 165 g einer 25 gewichtsprozentigen Natronlauge gerührt. Die organische Phase wurde abgetrennt, noch zweimal mit je 100 ml Wasser gewaschen und schliesslich einer Destillation bei 0,1 Torr unterworfen. Die zwischen 110 und 140 °C übergehende Fraktion wog 186 g, was einer Ausbeute an reinem Diolgemisch von 89 % der Theorie entsprach. Der Schmelzbereich des Gemisches betrug 45-82 °C.

Beispiel 5

In einer 1 l-Rührapparatur wurden bei 60 °C 252 g $\alpha$-$C_{18}$-Olefin (1 Mol) und 280 g 98 gewichtsprozentige Ameisensäure (6 Mol) vermischt und im Verlauf einer Stunde mit 52 g 85 gewichtsprozentigem Wasserstoffperoxid (1,3 Mol) versetzt. Das System blieb auch nach einer Nachreaktionszeit von 1 Stunde zweiphasig und wurde daher zunächst einmal einer Phasentrennung unterworfen. Dabei wurden 130 g wässrige Phase mit einem Ameisensäuregehalt von etwa 80 Gewichtsprozent abgetrennt. Aus der organischen Phase wurden bei vermindertem Druck 130 g Kopfprodukt abgetrieben. Der anfallende Sumpf wurde im Volumenverhältnis 1 : 2 mit Benzol vermischt und bei 70 °C mit 20 gewichtsprozentiger Natronlauge neutral eingestellt. Nach Phasentrennung wurde die organische Phase noch zweimal mit je 100 ml Wasser gewaschen und beim Abkühlen fraktioniert kristallisiert. Die Ausbeute an Reindiol mit einem Schmelzpunkt von 78,5-79,0 °C betrug 91,2 % der Theorie.

Beispiel 6

Als Ausgangsprodukt diente ein im Handel erhältliches $\alpha$-$C_{20-24}$-Olefingemisch, das nachstehende Zusammensetzung hatte :

| | |
|---|---|
| $\alpha$-$C_{18}$-Olefin | ca. 1 % |
| $\alpha$-$C_{20}$-Olefin | ca. 49 % |
| $\alpha$-$C_{22}$-Olefin | ca. 42 % |
| $\alpha$-$C_{24}$-Olefin | ca. 8 % |

Das Handelsprodukt hatte gemäss obiger Zusammensetzung ein mittleres Molgewicht von 306 und einen Schmelzpunkt von 32 °C. In einer 1 l-Rührapparatur wurden bei 60 °C 306 g Olefin mit 280 g Ameisensäure (98 %ig ; 6 Mol) vermischt und im Verlauf von 1 Stunde mit 48 g $H_2O_2$ (85 %ig ; 1,2 Mol) versetzt. Das System wurde eine weitere Stunde bei 60 °C gerührt und dann von der wässrigen Phase getrennt. Aus der verbleibenden organischen Phase wurde die überschüssig eingesetzte Ameisensäuremenge destillativ im Vakuum abgetrieben (bis 90 °C bei 12 Torr). Bei 90-100 °C wurde anschliessend das Sumpfprodukt mit 150 g Natronlauge (30 %ig) gerührt und danach (zur besseren Trennung der beiden Phasen) mit 600 ml Amylalkohol versetzt. Die wässrige Phase konnte heiss gut abgezogen werden und die organische Phase wurde mit 100 ml Wasser nachgewaschen. Aus der organischen Phase wurde das Lösungsmittel (Amylalkohol) destillativ entfernt. Das im Destillationsrückstand verbleibende Diol-Gemisch wog 340 g, hatte einen Restolefingehalt von 5 % und einen Erstarrungspunkt von 76 °C.

Beispiel 7

Als Ausgangsprodukt diente ein $\alpha$-Olefingemisch, das im Handel unter der Bezeichnung $C_{30^+}$-Olefin erhältlich ist. Das Gemisch soll einen Gehalt an $\alpha$-$C_{28}$-Olefin von 22 % haben und sich im übrigen aus $C_{30^-}$ und noch höheren Olefinen zusammensetzen. Aufgrund der Jodzahl wurde ein mittleres Molgewicht von 453 ermittelt, was wiederum einer mittleren Kohlenstoffzahl von ca. 32 entspricht.

1 Mol Olefingemisch $C_{30^+}$ wurde gemäss Beispiel 6 umgesetzt und aufgearbeitet, wobei lediglich die angewandte Menge 30 %iger Natronlauge auf 120 g gesenkt wurde. Gewonnen wurden 480 g eines Diolgemisches mit einem restolefingehalt von 10 % und einem Erstarrungspunkt von 90 °C.

Beispiel 8

2 Mol Cyclopenten (136,2 g ; $Kp_{760} = 44$ °C) wurden mit 8 Mol Ameisensäure (368 g ; 98 %ig) vermischt und in einer Rührapparatur (aufgesetzter Rückflusskühler) bei sogenannter « Siedekühlung » im Verlauf von 3 Stunden mit 2,4 Mol $H_2O_2$ (96,3 g ; 85 %ig) versetzt. Danach wurde das einphasig gewordene System weitere 2 Stunden bei 45 °C gerührt und im Anschluss daran destillativ von Ameisensäure und Reaktionswasser befreit (zum Schluss der Vakuumdestillation wurde bei 12 Torr eine Sumpftemperatur von ca. 90 °C erreicht).

Das Sumpfprodukt wurde mit 2,2 Mol NaOH (88 g gelöst in 200 ml $H_2O$) versetzt, gekühlt und

bei Zimmertemperatur dreimal hintereinander mit insgesamt 400 ml Äthylacetat extrahiert. Die vereinigten Extrakte wurden destillativ von Äthylacetat befreit. Bei der Destillation verblieb Cyclopentandiol als Rückstand (173 g), was einer 85 %igen Ausbeute entspricht. Durch weitere Extraktionen kann die Roh-Diol-Ausbeute erhöht werden. Durch Umkristallisation oder durch Destillation (Kp$_{12}$ = 125-130 °C) kann das Rohdiol weiter gereinigt werden.

Beispiel 9

1 Mol Neohexen (3,3-Dimethylbuten-1 ; 84 g ; Kp$_{760}$ = 41 °C) wurde mit 4 Mol Ameisensäure (184 g ; 98 %ig) vermischt und in einer Rührapparatur unter Siedekühlung im Verlauf von 1,5 Stunden mit 1,2 Mol H$_2$O$_2$ (48 g ; 85 %ig) versetzt. Das einphasig gewordene System wurde bei 50 °C eine weitere Stunde gerührt und danach destillativ von 2,7 Mol Ameisensäure befreit. Das Sumpfprodukt wurde mit 1,3 Mol NaOH ( = 52 g gelöst in 120 ml Wasser) verseift, gekühlt und fünfmal hintereinander mit je 100 ml Äthylacetat extrahiert. Aus den vereinigten Extrakten wurde das Äthylacetat abdestilliert. Als Sumpfprodukt wurden 94 g Roh-Diol erhalten (ca. 80 %ige Ausbeute), das durch Vakuumdestillation (Kp$_{15}$ = 105-110 °C) in reines Neohexandiol (Erstarrungspunkt = 38 °C) überführt wurde.

Beispiel 10

1 Mol Ölsaure (282 g) wurde mit 4 Mol Ameisensäure (98 %ig ; 184 g) vermischt und bei 50 °C mit 1,1 Mol H$_2$O$_2$ (70 %ig ; 54 g) im Verlauf von einer Stunde versetzt. Nach einer weiteren Stunde war der H$_2$O$_2$-Gehalt in dem einphasig gewordenen System auf 0,1 % abgesunken, das anschliessend destillativ von Ameisensäure und Wasser befreit wurde (bis 12 Torr und 90 °C im Sumpf). Das Sumpfprodukt wurde bei 70 °C mit 400 g NaOH (25 %ig) in Lösung gebracht. Aus dem dabei entstandenen Na-Salz der Dihydroxy-Stearinsäure wurde die Säure durch Versetzen mit 100 g HCl (conz.) in Freiheit gesetzt. In geschmolzenem Zustand wurde die ausgeschiedene Säure von der wässrigen Phase getrennt und mit 100 ml H$_2$O nachgewaschen. Gelöstes Wasser wurde durch Vakuumtrocknung entfernt. Es hinterblieben 301 g Roh-Dihydroxy-Stearinsäure entsprechend einer ca. 95 %igen Ausbeute. Durch Umkristallisation (z.B. aus Äthylacetat) kann die rohe Säure in ein Reinprodukt (Smp. 90/91 °C) überführt werden.

**Ansprüche**

1. Verfahren zur Hydroxylierung olefinisch ungesättigter Verbindungen mit 4 bis 36 Kohlenstoffatomen und über 40 °C liegenden Siedepunkten durch Umsetzung mit Ameisensäure und Wasserstoffperoxid bei erhöhter Temperatur und unter inniger Durchmischung und anschliessende Verseifung der enthaltenen Formiate, dadurch gekennzeichnet, dass man die Ameisensäure in Mengen von 2 bis 6 Mol pro Mol Doppelbindung einsetzt, Wasserstoffperoxid mit einer Konzentration von 35 bis 98 Gewichtsprozent verwendet und die Umsetzung bei einer Temperatur zwischen 40 und 80 °C vornimmt.

2. Verfahren nach Anspruch 1, dadurch gekennzeichnet, dass man ein Gemisch aus dem Olefin und der Ameisensäure vorlegt und das Wasserstoffperoxid im Verlaufe von 1 bis 4 Stunden zudosiert.

3. Verfahren nach Anspruch 1 oder 2, dadurch gekennzeichnet, dass man das Wasserstoffperoxid in einem molaren Überschuss von 5 bis 30 %, bezogen auf den Gehalt des eingesetzten Olefins an Doppelbindungen, einsetzt.

**Claims**

1. A process for the hydroxylation of olefinically unsaturated compounds having from 4 to 36 carbon atoms and boiling points above 40 °C, by reacting with formic acid and hydrogen peroxide at elevated temperature and with intimate intermixing and subsequent saponification of the resulting formates, characterised in that the formic acid is used in quantities of from 2 to 6 mols per mol of double bond, hydrogen peroxide is used in a concentration of from 35 to 98 % by weight and the reaction is carried out at a temperature of from 40 to 80 °C.

2. A process according to claim 1, characterised in that a mixture of the olefin and the formic acid is introduced and the hydrogen peroxide is metered in over a period of from 1 to 4 hours.

3. A process according to claim 1 or 2, characterised in that the hydrogen peroxide is used in a molar excess of from 5 to 30 %, based on the content of double bonds in the olefin used.

**Revendications**

1. Procédé d'hydroxylation de composés à insaturation oléfinique avec 4 à 36 atomes de carbone et des points d'ébullition se trouvant au-dessus de 40 °C, par réaction avec l'acide formique et le peroxyde d'hydrogène à température élevée et sous mélangeage intime et saponification ultérieure des formiates obtenus, caractérisé en ce que l'on utilise l'acide formique en des quantités de 2 à 6 mol par mol de doubles liaisons, que l'on utilise le peroxyde d'hydrogène avec une concentration de 35 à 98 % en poids et que l'on procède à la réaction à une température entre 40 et 80 °C.

2. Procédé selon la revendication 1, caractérisé en ce que l'on a au préalable un mélange de l'oléfine et de l'acide formique, et que l'on ajoute le peroxyde d'hydrogène sur 1 à 4 heures.

3. Procédé selon l'une des revendications 1 ou 2, caractérisé en ce que l'on utilise le peroxyde d'hydrogène en un excès molaire de 5 à 30 %, rapporté à la teneur de l'oléfine utilisée en doubles liaisons.